# EUROPEAN PATENT SPECIFICATION

(11) **EP 1 626 973 B1**
(45) Date of publication and mention of the grant of the patent: **20.10.2010**
(21) Application number: 04752844.3
(22) Date of filing: 20.05.2004
(51) Int. Cl.: C07D 487/04, A61K 31/407, A61P 29/00

(54) **(1S,5S)-3-(5,6-DICHLORO-3-PYRIDINYL)-3,6-DIAZABICYCLO[3.2.0]HEPTANE IS AN EFFECTIVE ANALGESIC AGENT**
(1S,5S)-3-(5,6-DICHLORO-3-PYRIDINYL)-3,6-DIAZABICYCLO[3.2.0]HEPTAN MIT ANALGETISCHER WIRKSAMKEIT
COMPOSE (1S,5S)-3-(5,6-DICHLORO-3-PYRIDINYL)-3,6-DIAZABICYCLO[3.2.0]HEPTANE UTILISE COMME AGENT ANALGESIQUE EFFICACE

(30) Priority: 27.05.2003 US 445555
(43) Date of publication of application: 22.02.2006
(73) Proprietor: ABBOTT LABORATORIES, Abbott Park, IL 60064-6008 (US)
(72) Inventor: BUCKLEY, Michael, J., McHenry, IL 60050 (US); JI, Jianguo, Libertyville, IL 60048 (US)
(74) Representative: Modiano, Micaela Nadia
(86) International application number: PCT/US2004/015905
(87) International publication number: WO 2004/106342

(56) References cited:
- WO-A-01/81347

## Description

### FIELD OF THE INVENTION

The present invention is directed to (1S,5S)-3-(5,6-dichloro-3-pyridinyl)-3,6-diazabicyclo[3.2.0]heptane and its use to treat pain, in particular, neuropathic pain.

### BACKGROUND OF THE INVENTION

The search for potent and effective analgesics continues to be a significant research goal in the medical community. A substantial number of medical disorders and conditions produce pain as part of the disorder or condition. Relief of this pain is a major aspect of ameliorating or treating the overall disorder or condition. Pain and the possible allievation thereof is also attributable to the individual patient's mental condition and physical condition.

Opioid and non-opioid drugs are the two major classes of analgesics (A. Dray and L. Urban, Ann. Rev. Pharmacol. Toxicol., 36:253-280, (1996)). Opioids, such as morphine, act at opioid receptors in the brain to block transmission of the pain signals in the brain and spinal cord (N.I. Cherney, Drug, 51:713-737, (1996)). Non-opioids such as non-steroid anti-inflammatory agents (NSAIDs) typically, but not exclusively, block the production of prostaglandins to prevent sensitization of nerve endings that facilitate the pain signal to the brain (Dray, et al., Trends in Pharmacol. Sci., 15:190-197, (1994); T.J. Carty and A. Marfat, "COX-2 Inhibitors. Potential for reducing NSAID side-effects in treating inflammatory diseases", Emerging Drugs: Prospect for Improved Medicines. (W. C. Bowman, J.D. Fitzgerald, and J.B. Taylor, eds.), Ashley Publications Ltd., London, Chap. 19., pp. 391-411).

Certain compounds, with primary therapeutic indications other than analgesia, have been shown to be effective in some types of pain control. These are classified as analgesic adjuvants, and include tricyclic antidepressants (TCAs) and some anticonvulsants such as gabapentin (Williams et al., J. Med. Chem., 42:1481-1500 (1999)). They are used increasingly for treatment of pain, especially for pain resulting from nerve injury due to trauma, radiation, or disease.

(1S,5S)-3-(5,6-Dichlono-3-pyridinyl)-3,6-diazabicyclo[3.2.0]heptane is a novel compound that has utility in treating pain and disorders associated with the nicotinic acetylcholine receptor (nAChR). (1S,5S)-3-(5,6-Dichloro-3-pyrldinyl)-3,6-diazabicyclo[3.2.0]heptane may also have utility when administered in combination with an opioid such as morphine, a non-steroid anti-inflammatory agent such as aspirin, a tricyclic antidepressant, or an anticonvulsant such as gabapentin or pregabalin for treating pain and disorders associated with the nicotinic acetylcholine receptor.

WO 01-81347 discloses diazabicyclo[3.2.0]heptanes that are analgesic agents.

### SUMMARY OF THE INVENTION

The present invention discloses (1S,5S)-3-(5,6-dichloro-3-pyridinyl)-3,6-diazabicyclo[3.2.0]heptane or a pharmaceutically acceptable salt thereof and its use to treat pain, in particular, neuropathic pain.

### DETAILED DESCRIPTION OF THE INVENTION

In its principle embodiment, the present invention discloses (1S,5S)-3-(5,6-dichloro-3-pyridinyl)-3,6-diazabicyclo[3,2.0]heptane or a pharmaceutically acceptable salt thereof.

In another embodiment, the present invention relates to (1S,5S)-3-(5,6-dichloro-3-pyridinyl)-3,6-diazabicyclo[3.2.0]heptane or a pharmaceutically acceptable salt thereof. For use in treating pain including, but not limited to, neuropathic pain by administering to a mammal a therapeutically effective amount of said (1S,5S)-3-(5,6-dichloro-3-pyridinyl)-3,6-diazabicyclo[3.2.0]heptane or a pharmaceutically acceptable salt thereof.

In another embodiment, the present invention relates to (1S,5S)-3-(5,6-dichloro-3-pyridinyl)-3,6-diazabicyclo[3.2.0]heptane or a pharmaceutically acceptable salt thereof. For use in treating pain including, but not limited to, neuropathic pain comprising administering to a mammal a therapeutically effective amount of said (1S,5S)-3-(5,6-dichloro-3-pyridinyl)-3,6-diazabicyclo[3.2.0]heptane or a pharmaceutically acceptable salt thereof in combination with an opioid including, but not limited to morphine.

In another embodiment, the present invention relates to (1S,5S)-3-(5,6-dichloro-3-pyridinyl)-3,6-diazabicyclo[3.2.0]heptane or a pharmaceutically acceptable salt thereof. For use in treating pain including, but not limited to, neuropathic pain by administering to a mammal a therapeutically effective amount of said (1S,5S)-3-(5,6-dichloro-3-pyridinyl)-3,6-diazabicyclo[3.2.0]heptane or a pharmaceutically acceptable salt thereof in combination with a non-steroid anti-inflammatory agent including, but not limited to aspirin.

In another embodiment, the present invention relates to (1S,5S)-3-(5,6-dichloro-3-pyridinyl)-3,6-diazabicyclo[3.2.0]heptane or a pharmaceutically acceptable salt thereof. For use in treating pain including, but not limited to, neuropathic pain by administering to a mammal a therapeutically effective amount of said (1S,5S)-3-(5,6-dichloro-3-pyridinyl)-3,6-diazabicyclo[3.2.0]heptane or a pharmaceutically acceptable salt thereof in combination with an anticonvulsant including, but not limited to, gabapentin or pregabalin.

In another embodiment, the present invention relates to (1S,5S)-3-(5,6-dichloro-3-pyridinyl)-3,6-diazabicyclo[3.2.0]heptane or a pharmaceutically acceptable salt thereof. For use in treating pain including, but not limited to, neuropathic pain by administering to a mammal a therapeutically effective amount of said (1S,5S)-3-(5,6-dichloro-3-pyridinyl)-3,6-diazabicyclo[3.2.0]heptane or a pharmaceutically acceptable salt thereof in combination with a tricyclic antidepressant.

In another embodiment, the present invention relates to (1S,5S)-3-(5,6-dichloro-3-pyridinyl)-3,6-diazabicyclo[3.2.0]heptane or a pharmaceutically acceptable salt thereof. For use in treating Alzheimer's disease, Parkinson's disease, memory dysfunction, Tourette's syndrome, sleep disorders, attention deficit hyperactivity disorder, neurodegeneration, inflammation, neuroprotection, anxiety, depression, mania, schizophrenia, anorexia and other eating disorders, AIDS-induced dementia, epilepsy, urinary incontinence, substance abuse, smoking cessation or inflammatory bowel syndrome, by administering to a mammal a therapeutically effective amount of said (1S,5S)-3-(5,6-dichloro-3-pyridinyl)-3,6-diazabicyclo[3.2.0]heptane or a pharmaceutically acceptable salt thereof.

In another embodiment, the present invention relates to pharmaceutical compositions comprising (1S,5S)-3-(5,6-dichloro-3-pyridinyl)-3,6-diazabicyclo[3.2.0]heptane or a pharmaceutically acceptable salt thereof in combination with a pharmaceutically acceptable carrier.

### Preparation of (1S,5S)-3-(5,6-Dichloro-3-pyridinyl)-3,6-diazablcyclo[3.2.0]heptane

### Example 1

### tert-butyl (1R,5S)-3,6-diazabicyclo[3.2.0]heptane-6-carboxylate

### Example 1A

### benzyl 2,2-dimothoxyothylcarbamate

Benzyl chloroformate (231.3 g, 1.3 mol) was added gradually to a mixture of aminoacetaldehyde dimethyl acetal (152.0 g, 1.3 mol) in toluene (750 mL) and aqueous NaOH (72.8 g, 1.82 mol; in 375 mL of water) at 10-20°C. After the addition was completed, the mixture was stirred at ambient temperature about 4 hours. The organic layer was separated, washed with brine (2 x 100 mL) and concentrated to provide the title compound. ¹H NMR (CDCl₃, 300 MHz) δ 3.33 (t, J=6.0Hz, 2H), 3.39 (s, 6H), 4.37 (t, J=6.0Hz, 1H), 5.11 (s, 2H), 7.30 (m, 5H); MS (DCI/NH₃) m/z 257 (M+NH₄)⁺, 240 (M+H)⁺.

### Example 1B

### benzyl allyl(2,2-dimethoxyethyl)carbamate

The product of Example 1A (281.0 g, 1.18 mol) in dry toluene (1.0 L) was treated with powdered KOH (291.2 g, 5.20 mol) and triethylbenzylammonium chloride (4.4 g, 0.02 mol). A solution of allyl bromide (188.7 g, 1.56 mol) in toluene (300 mL) was then added dropwise over 1 hour at 20-30 °C. The mixture was stirred overnight at room temperature and then water (300 mL) was added over 20 minutes at 20-30 °C. The layers were separated and the aqueous phase was extracted with toluene ( 2 x 300 mL). The organic phases were combined, washed with brine (2 x 100 mL), dried (K₂CO₃), filtered and the filtrate concentrated to provide the title compound. ¹H NMR (MeOH-d₄, 300 MHz) δ 3.32 (s, 3H) 3.37 (m, 5H), 3.97 (d, J=5.4 Hz, 2H), 4.40-4.50 (m, 1H), 5.15 (m, 4H), 5.75 (m, 1H), 7.23 (m, 5H); MS (DCI/NH₃) m/z 297 (M+NH₄)⁺, 280 (M+H)⁺.

### Example 1C

### benzyl allyl(2-oxoethyl)carbamate

The product of Example 1B (314.0 g, 1.125 mol) was treated with formic acid (88%, 350 mL) at room temperature and allowed to stir for 15 hours. Most of the formic acid was removed by concentration under reduced pressure at 40-50°C. The residue was extracted with ethyl acetate (3 x 500 mL). The extracts were combined and washed with brine until the wash had a pH = 6-7. The organic phase was concentrated to provide the title compound. ¹H NMR (CDCl₃, 300 MHz) δ 3.20 (m, 1H), 3.97 (m, 2H), 4.10 (m, 1H), 5.10 (m, 4H), 5.75 (m, 1H), 7.45 (m, 5H), 9.50 (d, J=6.4 Hz, 1H); MS (DCI/NH₃) m/z 234 (M+H)⁺.

### Example 1D

### benzyl allyl[2-(hydroxyimino)ethyl]carbamate

The product of Example 1C (260 g, 1.115 mol) in acetonitrile (1.5 L) was treated with sodium acetate trihydrate (170.6 g, 4.41 mol) in distilled water (750 mL) and NH₂OH hydrochloride (98.0 g, 4.41 mol) under N₂. The mixture was stirred at room temperature for about 20 hours. The volatiles were removed under reduced pressure and the residue was extracted with ethyl acetate (2 x 750 mL). The combined organic phases were washed with brine until the wash had a pH = 7. The organic phase was concentrated to provide the title compound. ¹H NMR (MeOH-d₄, 300 MHz) δ 3.94 (m, 2H), 3.98 (d, J=5.5Hz, 1H), 4.17 (d, J=4.4 Hz, 1H), 5.30 (m, 4H), 5.60 (m, 1H), 7.40 (m, 5H). MS (DCI/NH₃) m/z 266M+NH₄)⁺, 249 (M+H)⁺.

### Example 1E

### benzyl (cis)-3-amino-4-(hydroxymethyl)-1-pyrrolidinecarboxylate

A solution of the product of Example 1D (240 g, 0.97 mol) in xylene (1.0 L) was heated at reflux under N₂ for about 10 hours. The resulting brown solution was cooled to 10-15 °C and acetic acid (1.0 L) was added under N₂. Zinc powder (100 g, 1.54 mol) was added gradually, and the gray mixture was stirred at room temperature for 3 hours. The mixture was filtered and water (1.0 L) was added to the filtrate. The filtrate was stirred for 10 minutes and the organic layer was separated. The aqueous phase was washed well with xylenes (4 x 400 mL) and then concentrated under reduced pressure to a volume of approximately 200 mL. This residue was basified to pH 9-10 by addition of saturated aqueous Na₂CO₃. The precipitated white solid was removed by filtration and the filtrate was extracted with CHCl₃ (3 x 600 mL). The combined organic phases were washed with saturated Na₂CO₃ solution (2 x 50 mL) and dried over anhydrous Na₂CO₃. The mixture was filtered through a short column of diatomaceous earth and the filtrate was concentrated to provide the title compound. ¹H NMR (MeOH-d₄, 300 MHz) δ 2.40 (m, 1H), 3.30 (m, 2H), 3.80-3.50 (m, 5H), 5.10 (s, 2H), 7.35 (m, 5H); MS (DCUNH₃) m/z 251 (M+H)⁺.

### Example 1F

### benzyl (4aS.7aS)-2,2-dimethylhexahydropyrrolo[3,4-d][1,3]oxazine-6(4H)-carboxylate (R)-Mandelate

The product of Example 1E (140g, 0.56 mol) in dry acetone (150 mL) was treated with 2-methoxypropene (55 mL, 0.57 mol) at room temperature overnight. The reaction mixture was concentrated under reduced pressure and the residue was dissolved in dry acetone (750 mL). (R)-Mandelic acid (85 g, 0.56 mol) was added and the solution was stirred at room temperature for 48 hours. The precipitate was isolated by filtration and dried under reduced pressure to provide the title compound as a solid. ¹H NMR (MeOH-d₄, 300 MHz) δ 1.20-1.40 (m, 3H), 2.09 (s, 3H), 3.30 (m, 1H), 3.48-3.75 (m, 6H), 4.20 (m, 1H), 5.10 (m, 3H), 7.25-7.52 (m, 10H); MS (DCI/NH₃) m/z 291 (M+H)⁺.

### Example 1G

### benzyl (3S,4S)-3-[(tert-butoxycarbonyl)amino]-4-(hydroxymethyl)-1-pyrrolidinecarboxylate

The product of Example 1F (56 g, 127 mmol) in ethanol (50 mL) was treated with 5% aqueous H₂SO₄ (100 mL) at room temperature and allowed to stir for 16 hours. The mixture was basified to pH ∼ 10 with 20% aqueous NaOH (50 mL) and then the mixture was treated with di-tert-butyl dicarbonate (41.5 g, 190 mmol) in ethanol (50 mL) at 10-20 °C. After stirring at room temperature for 4 hours, the ethanol was removed under reduced pressure and the residue was extracted with ethyl acetate (3 x 500 mL). The combined organic phases were washed with brine (2 x 100 mL) and concentrated to provide the title compound. The enantiopurity of the title compound was determined to be greater than or equal 99% enantiomeric excess by HPLC (HPLC conditions: Chiracel AD column; ethanol/hexanes=20/80, flow rate, 1.0 mL/minute; uv 220 nm; retention time 10.8 minutes). ¹H NMR (MeOH-d₄, 300 MHz) δ 1.46 (s, 9H), 2.50 (m, 1H), 3.25 (m, 1H), 3.40 (m, 1H), 3.50-3.75 (m, 4H), 4.20 (m, 1H), 5.10 (s, 2H), 7.35 (m, 5H); MS (DCI/NH₃) m/z 368 (M+NH₄)⁺, 351 (M+H)⁺.

### Example 1H

### benzyl (3S,4S)-3-f(tert-butoxycarbonyl)aminol-4-{[(methylsulfonyl)oxy]methyl}-1-pyrrolidinecarboxylate

The product of Example 1G (43.7 g, 125 mmol) and triethylamine (25.2 g, 250 mmol) in CH₂Cl₂ (600 mL) were treated with methanesulfonyl chloride (12.6 mL, 163 mmol) over 30 minutes at -10 °C. The solution was allowed to warm to room temperature over 1 hour and quenched with water (100 mL). The layers were separated and the aqueous phase was extracted with CH₂Cl₂ (2 x 400 mL). The combined organic phases were washed with brine (2 x 100 mL), dried over Na₂SO₄, filtered, and the filtrate concentrated to provide the title compound. ¹H NMR (CDCl₃, 300 MHz) δ 1.46 (s, 9H), 2.80 (m, 1H), 3.08 (s, 3H), 3.40(m, 2H), 3.70 (m, 2H), 4.10 (m, 1H), 4.40 (m, 2H), 4.75 (m, 1H), 5.16 (s, 2H), 7.30 m, 5H); MS (DCT/NH₃) m/z 446 (M+NH₄)⁺,429 (M+H)⁺.

### Example 1I

### benzyl (3S,4S)-3-amino-4-{[(methylsulfonyl)oxy]methy}-1-pyrrolidinecarboxylate trifluroacetate

The product of Example 1H (43.7 g, 125 mmol) in CH₂Cl₂ (150 mL) was treated with trifluoroacetic acid (50 mL) at room temperature and allowed to stir for 1 hour. The mixture was concentrated under reduced pressure to give the title compound. ¹H NMR (CDCl₃, 300 MHz) δ 2.80 (m, 1H), 3.15 (s, 3H), 3.40(m, 1H), 3.70 (m, 3H), 4.10 (m, 1H), 4.05 (m, 1H), 4.44 (m, 2H), 5.16 (s, 2H), 7.30-7.50(m, 5H); MS (DCI/NH₃) m/z 329 (M+H)⁺.

### Example 1J

### benzyl (1S,5S)-3,6-diazabicyclo[3.2.0]heptane-3-carboxylate

The product of Example 1I was dissolved in ethanol (250 mL) and basified to pH ∼12 with 25% aqueous NaOH. The mixture was warmed to 60 °C for 1.5 hours. The reaction mixture was allowed to cool to room temperature and used in the next step without further purification. An analytical sample was removed (∼1mL) and concentrated under reduced pressure. The residue was extracted with CHCl₃ (2 x 5 mL). The extracts were combined, washed with brine (3 x 2 mL) and then passed through a short column of diatomaceous earth. The filtrate was concentrated to provide an analytical amount of the title compound. ¹H NMR (MeOH-d₄, 300 MHz) δ 3.30-3.16 (m, 3H), 3.36 (m, 1H), 3.82 (m, 3H), 4.55 (m, 1H), 5.20 (s, 2H), 7.36 (m, 5H); MS (DCI/NH₃) m/z 250 (M+NH₄)⁺, 233 (M+H)⁺.

### Example 1K

### 3-benzyl, 6-tert-butyl-(1R,5S)-3,6-diazabicyclo[3.2.0]heptane-3,6-dicarboxylate

The solution of Example 1J was slowly added to di-tert-butyl dicarbonate (40.9 g, 188 mmol) in ethanol (50 mL) over 30 minutes at room temperature. The mixture was stirred at room temperature for additional 0.5-1 hours. The reaction mixture was concentrated under reduced pressure. The residue was extracted with ethyl acetate (3 x 500 mL). The ethyl acetate extracts were combined, washed with brine (3 x 50 mL), stirred with KHSO₄ (5%, 100 mL) for 10 minutes and the phases separated. The organic layer was washed with brine (3 x 50 mL) and passed through a short column of diatomaceous earth. The filtrate was concentrated to provide the title compound which was used in the next step without further purification. ¹H NMR (MeOH-d₄, 300 MHz) δ 1.4 (s, 9H), 3.10 (m, 2H), 3.30 (m, 1H), 3.45 (m, 1H), 3.90 (d, J=12.2 Hz, 1H), 4.06 (m, 2H), 4.66 (dd, J=6.4, 2.0 Hz, 1H), 5.16 (s, 2H), 7.36 (m, 5H); MS (DCI/NH₃) m/z 333 (M+H)⁺.

### Example 1L

### tert-butyl (1R,5S)-3,6-diazabicyrclo[3.2.0]heptane-6-carboxylate

The product of Example 1K (40.0 g, 0.120 mol) was dissolved in methanol (400 mL) and treated with Pd/C (10 wt.%, 4.0g) under H₂ at room temperature for 10 hours. The reaction mixture was filtered through a short column of diatomaceous earth and the filtrate was concentrated to provide the title compound. ¹H NMR (MeOH-d₄, 300 MHz) δ 1.43 (s, 9H), 2.47(dd, J=12.6, 3.8 Hz, 1H), 2.62 (dd, J=12.2, 5.7 Hz, 1H), 2.96 (m, 1H), 3.05 (d, J=12.2 Hz, 1H), 3.22 (d, J=12.5 Hz, 1H), 3.45 (m, 1H), 3.95 (m, 1H), 4.63 (dd, J=6.1, 3.7 Hz, 1H); MS (DCI/NH₃) m/z 199 (M+H)⁺.

### Example 2

### 5-bromo-2,3-dichloropyridine

### Example 2A

### 3-chloro-5-nitro-2-pyridinol

A 5L flask with mechanical stirrer, thermocouple, and addition funnel was charged with 2-hydroxy-5-nitropyridine (200 g, 1.43 mol, Aldrich) and concentrated HCl (890 mL). The mixture was warmed to 50 - 55 °C and a solution of KClO₃ (61.3 g, 0.5 mol) in water (850 mL) was added dropwise over 75 minutes maintaining the reaction temperature at 55 - 59 °C. Following complete addition, the reaction mixture was cooled in an ice-water bath to an internal temperature of <6 °C and then filtered. The filter cake was washed with cold water (700 mL) and dried under vacuum at 50 °C for 12 hours to provide the title compound. ¹H NMR (CDCl₃, 300 MHz) δ 7.43 (d, J=3 Hz, 1H), 7.59 (d, J=3 Hz, 1H).

### Example 2B

### 2,3-dichloro-5-nitropyridine

A 2L flask with mechanical stirrer and thermocouple was charged with POCl₃ (200 g, 1.30 mol). The flask was cooled in an ice bath to an internal temperature of 0-5 °C as quinoline (84 g, 0.65 mol) was added. The product of Example 2A (227 g, 1.30 mol) was added in portions, so as to maintain the reaction temperature below 10 °C. The cold bath was removed, and the mixture was warmed to 120 °C for 90 minutes. The temperature was decreased to 100 °C and the reaction mixture was quenched by addition of water (500 mL) maintaining the internal temperature between 100-110 °C. After complete addition, the mixture was cooled in ice to 0-5 °C for 1 hour and filtered. The filter cake was washed with cold water and dried under vacuum at 40 °C to provide the title compound. ¹H NMR (CDCl₃, 300 MHz) δ 8.39 (d, J=3 Hz, 1H), 9.16 (d, J=3 Hz, 1H).

### Example 2C

### 5-amino-2.3-dichloropyridine

Anhydrous SnCl₂ (300 g, 1.58 mol) and concentrated HCl (350 mL) were charged to a 5L flask with mechanical stirrer and thermocouple. The flask was cooled in ice and the product of Example 2B (100 g, 0.518 mol) was added in portions maintaining the temperature below 65 °C. After the addition was complete, the cold bath was removed, and the mixture was stirred for 2 hours at ambient temperature. The mixture was cooled in ice as 25% aqueous NaOH (1000 mL) was added to bring the mixture to pH >10. The mixture was extracted with CH₂Cl₂ (1 x 600 mL, 2 x 400 mL) and the combined extracts were washed with brine (200 mL), dried (MgSO₄), and concentrated under vacuum. The residual solid was crystallized from a mixture of water (500 mL) and ethanol (100 mL) to provide the title compound as a solid. ¹H NMR (CDCl₃, 300 MHz) δ 3.80 (br s, 2H), 7.10 (d, J=3 Hz, 1H), 7.77 (d, J=3 Hz, 1H); MS (DCI/NH₃) m/z 180/182/184 (M+NH₄)⁺ 163/165/167 (M+H)⁺.

### Example 2D

### 5-bromo-2,3-dichloropyridine

A 5L flask with mechanical stirrer, thermocouple, and addition funnel was charged with the product of Example 2C (70 g, 429 mmol) and 48% HBr_{aq} (240 mL). The suspension was maintained at 0-5 °C as a solution of NaNO₂ (32.0g, 464 mmol) in water (100 mL) was added dropwise over 1 hour. Additional water (200 mL) was added and the mixture was stirred for 10 minutes at 0-5 °C. The mixture was treated with CuBr (32.6 g, 227 mmol) in portions over 20 minutes followed by additional water to maintain a fluid reaction mixture. The mixture was allowed to warm to room temperature and diluted with water. The mixture was distilled at ambient pressure, until the distillate ran clear (1.5 L collected). The distillate was extracted with EtOAc (3 X 500 mL) and the combined extracts were washed with brine (100 mL), dried (MgSO₄), and concentrated to provide 5-bromo-2,3-dichloropyridine as a solid. ¹H NMR (CDCl₃, 300 MHz) δ 7.94 (d, J=3 Hz, 1H), 8.38 (d, J=3 Hz, 1H).

### Example 3

### (1S,5S)-3-(5,6-dichloro-3-pyridinyl)-3,6-diazabicyclo[3.2.0]heptane (L)-tartrate

### Example 3A

### tert-butyl (1R,5S)-3-(5,6-dichloro-3-pyridinyl)-3,6-diazabicyclo[3.2.0]hetapne-6-carboxylate

A 1L flask with mechanical stirrer was charged with a solution of tert-butyl (1R,5S)-3,6-diazabicyclo[3.2.0]heptane-6-carboxylate (10.0 g, 50 mmol, product of Example 1L) and 5-bromo-2,3-dichloropyridine (14.0 g, from Example 2D) in toluene (400 mL). The flask was evacuated and purged three times with nitrogen. Xantphos (1.74 g, 3 mmol, Strem Chemicals), Pd₂(dba)₃ (916 mg, 1 mmol, Strem Chemicals) and sodium tert-butoxide (7.20 g, 75 mmol) were added successively to the flask against a purge of nitrogen gas. The flask was again evacuated and purged with nitrogen (3 times) and the mixture heated to 85-90 °C under N₂. After 2 hours, the reaction was cooled to room temperature, diluted with ethyl acetate (1000 mL) and water (200 mL), and stirred for 5 minutes. The organic phase was separated, washed with brine (200 mL), dried (MgSO₄), filtered through Celite^{®}, and the filtrate concentrated under vacuum to provide the title compound which was used in the next step without further purification. ¹H NMR (MeOH-d₄, 300 MHz) δ 1.45 (s, 9H), 2.94 (dd, J=11.6, 4.4 Hz, 1H), 3.04 (dd, J=10.2, 6.4 Hz, 1H), 3.3 (m, 1H), 3.58 (m, 1H), 3.78 (d, J=10.5 Hz, 1H), 3.90 (d, J=10.8 Hz, 1H), 4.05 (m, 1H), 4.83 (m, 1H) 7.39 (d, J=2.7 Hz, 1H), 7.84 (d, J=2.7 Hz, 1H); MS (DCI/NH₃) m/z 344/346/348 (M+H)⁺.

### Example 3B

### (1S,5S)-3-(5,6-dichloro-3-pyridinyl)-3,6-diazabicyclo[3.2.0]heptane p-toluenesulfonate

The product of Example 3A (23.2 g) was dissolved in ethyl acetate (250 mL) and p-toluenesulfonic acid monohydrate (11.4 g, 60 mmol) was added. The solution was warmed to reflux and stirred for 90 minutes, cooled to room temperature, and allowed to stand for 12 hours to complete precipitation. The solid was isolated by filtration and dried to provide the title compound. mp 174-178 °C; [α]_{D}²⁰=-20.0°(MeOH, 0.105); ¹H NMR (MeOH-d₄, 300 MHz) δ 2.36 (s, 3H), 3.06 (dd, J=10.5, 6.1 Hz, 1H), 3.17 (dd, J=12.2, 4.8 Hz, 1H), 3.50 (m, 1H), 3.72 (dd, J=11.2, 5.4 Hz, 1H), 3.90 (d, J=10.5 Hz, 1H), 4.10 (d, J=12.6 Hz, 1H), 4.25 (dd, J=11.2, 9.8 Hz, 1H), 5.05 (dd, J=6.7, 5.1 Hz, 1H) 7.22 (d, J=8.1 Hz, 2H), 7.52 (d, J=2.7 Hz, 1H), 7.69 (d, J=8.1 Hz, 2H), 7.95 (d, J=2.7 Hz, 1H); MS (DCI/NH₃) m/z 244/246/248 (M+H)⁺.

### Example 3C

### (1S,5S)-3-(5,6-dichloropyridin-3-yl)-3,6-diaza-bicyclor[3.2.0]heptane

The product of Example 3B (33 g, 79 mmol) was stirred in 330 mL of 5% NaOH in water for 10 minutes and extracted with CHCl₃:i-PrOH (10:1) (4 x 500 mL). The extract were combined, washed with brine (2 x 100 mL), and concentrated to give the title compound as a solid. ¹H NMR (MeOH-d₄, 300 MHz) δ 3.04 (dd, J=10.9, 4.8 Hz, 1H), 3.11 (dd, J=10.2, 6.8 Hz, 1H), 3.26 (dd, J=8.8, 4.4 Hz, 1H), 3.38 (m, 1H), 3.73 (t, J=11.2 Hz, 2H), 3.84 (t, J=8.1 Hz, 1H), 4.55 (dd, J=6.8, 4.8 Hz, 1H), 7.37 (d, J=3.1 Hz, 1H), 7.84 (d, J=2.7 Hz, 1H); MS (DCI/NH₃) m/z 244/246/248 (M+H)⁺.

### Example 3D

### (1S,5S)-3-(5,6-dichloro-3-pyridinyl)-3,6-diazabicyclo[3.2.0]heptane (L)-tartrate

The product from Example 3C (12.0 g, 50 mmol) in MeOH (400 mL) was heated to 65 °C and treated with (L)-tartaric acid (9.0 g, 60 mmol) in MeOH (60 mL) dropwise. After complete addition, the mixture was stirred at reflux for 2 hours and then allowed to cool to room temperature. After stirring at room temperature for 10 hours, the mixture was filtered and the filter cake washed with chilled methanol (10 mL). The solid was dried under vacuum to provide the title compound. mp 210-212 °C (decomp); [α]_{D}²⁰ = -27.02° (MeOH, 0.105); ¹H NMR (MeOH-d₄, 300 MHz) δ 3.12 (dd, J=10.9, 6.1 Hz, 1H), 3.22 (dd, J=12.9, 5.1 Hz, 1H), 3.54 (m, 1H), 3.76 (dd, J=11.6, 5.1 Hz, 1H), 3.87 (d, J=10.9 Hz, 1H), 4.10 (d, J=12.6 Hz, 1H), 4.31 (dd, J=11.2, 8.5 Hz, 1H), 4.77 (s, 2H), 5.13 (dd, J=7.2, 5.1 Hz, 1H) 7.54 (d, J=2.7 Hz, 1H), 7.90 (d, J=2.7 Hz, 1H); MS (DCI/NH₃) m/z 244/246/248 (M+H)⁺.

### In Vitro Data

### Determination of Binding Potency

(1S,5S)-3-(5,6-Dichloro-3-pyridinyl)-3,6-diazabicyclo[3.2.0]heptane was subjected to an vitro assay against the nicotinic acetylcholine receptor as described below.

Binding of [³H]-cytisine ([³H]-CYT) to neuronal nicotinic acetylcholine receptors was accomplished using crude synaptic membrane preparations from whole rat brain (Pabreza et al., Molecular Pharmacol., 1990, 39:9). Washed membranes were stored at -80 °C prior to use. Frozen aliquots were slowly thawed and resuspended in 20 volumes of buffer (containing: 120 mM NaCl, 5 mM KCl, 2 mM MgCl₂, 2 mM CaCl₂ and 50 mM Tris-Cl, pH 7.4 @4 °C). After centrifuging at 20,000x g for 15 minutes, the pellets were resuspended in 30 volumes of buffer.

Each test compound was dissolved in water to make 10 mM stock solutions, diluted (1:100) with buffer (as above), and further taken through seven serial log dilutions to produce test solutions from 10⁻⁵ to 10⁻¹¹ M.

Homogenate (containing 125-150 µg protein) was added to triplicate tubes containing the range of concentrations of test compound described above and [³H]-CYT (1.25 nM) in a final volume of 500 µL. Samples were incubated for 60 minutes at 4 °C, then rapidly filtered through Whatman GFB filters presoaked in 0.5% polyethyleneimine using 3 x 4 mL of ice-cold buffer. The filters are counted in 4 mL of Ecolume® (ICN). Nonspecific binding was determined in the presence of 10 µM (-)-nicotine and values were expressed as a percentage of total binding. The IC₅₀ value was determined with the RS-1 (BBN) nonlinear least-squares curve-fitting program and the IC₅₀ value was converted to a Ki value using the Cheng and Prusoff correction (Kᵢ=IC₅₀/(1+[ligand]/Kd of ligand). The Ki value for (1S,5S)-3-(5,6-dichloro-3-pyridinyl)-3,6-diazabicyclo[3.2.0]heptane was determined to be 0.10 nM.

### In Vivo Data

### Determination of Analgesic Effect

Male Sprague Dawley rats (80-100 g) were purchased from Charles River (Portage, MI). Prior to surgery, animals were group-housed and maintained in a temperature regulated environment (lights on between 7:00 a.m. and 8:00 p.m.). Following nerve ligation surgery, animals were group housed. Rats had access to food and water ad libitum.

The L5 and L6 spinal nerves of anesthesized rats were tightly ligated in the manner described previously by S.H. Kim and J.M. Chung, PAIN 50:355 (1992). Briefly, an incision was made on the dorsal portion of the hip and the muscle was blunt dissected to reveal the spinal processes. The L6 transverse process was removed, and the left L5 and L6 spinal nerves were tightly ligated with 5.0 braided silk suture. The wound was cleaned, the membrane sewn with 4.0 dissolvable Vicryl suture and the skin closed with wound clips.

For the assessment of neuropathic pain, mechanical allodynia in the affected paw of animals that had undergone spinal nerve ligation was evaluated using von Frey filaments. As described previously by S.R. Chaplan, F.W. Bach, J.W. Pogrel, J.M. Chung, and T.L. Yaksh, "Quantitative assessment of tactile allodynia in the rat paw" J. Neurosci. Meth., 53:55-63 (1994) two weeks following surgery, rats were acclimated to the testing box that was constructed of plexiglass with a wire mesh floor to allow access to the planter surface of the hindpaws. Using the Dixons Up-Down method, a baseline level of allodynia was determined to have a withdrawal threshold of ≤ 4 g of pressure. (1S,5S)-3-(5,6-Dichloro-3-pyridinyl)-3,6-diazabicyclo[3.2.0]heptane, administered intraperitoneally 15 minutes before testing, caused a dose-dependent increase in the withdrawal threshold up to a maximum effect of 15 g. The EC₅₀ was determined to be 1 µmol/kg.

### Determination of Side Effect Liability

Cells of the IMR-32 human neuroblastoma clonal line (ATCC, Rockville, MD) were maintained in a log phase of growth according to established procedures by R.J. Lukas, "Expression of ganglia-type nicotinic acetylcholine receptors and nicotinic ligand binding sites by cells of IMR-32 human neuroblastoma clonal line" J. Pharmacol. Exp. Ther. 265:294-302 (1993). Cells were plated out at a density of 1×10⁶ cells per well on black-walled, clear-bottomed, 96-well plates (Costar, Cambridge, MA) and used approximately 72 hours after plating. All plates were coated with polyethylenimine to aid in the adherence of the cells to the plate.

Changes in the intracellular Ca²⁺ content of IMR-32 cells were measured using the calcium chelating dye Fluo-4 (Molecular Probes, Eugene, OR) in conjunction with a Fluorescent Imaging Plate Reader (Molecular Devices, Sunnyvale, CA). The cell permeant acetoxymethyl (AM) ester form of Fluo-3 was prepared to a concentration of 1 mM in anhydrous DMSO and 10% pluronic acid. The dye was then diluted to a final concentration of 4 mM in growth media and placed on the cells for 1 hour at 37 °C. Black-walled 96-well plates were utilized to reduce light scattering. The unincorporated dye was removed from the cells by excessive washing with the assay buffer (HETES buffer, 20 mM Hepes, 120 mM NaCl, 5 mM KCl, 1 mM MgCl₂, 5 mM glucose, 500 mM atropine, and 5 mM CaCl₂). After addition of various concentrations of (1S5S)-3-(5,6-Dichloro-3-pyridinyl)-3,6-diazabicyclo[3.2.0]heptane, the Ca²⁺ dynamics were observed in the Fluorescent Imaging Plate Reader (FLIPR) apparatus equipped with an Argon laser (wavelength, 480 nm), an automated 96 channel pipettor and a CCD camera. The intensity of the fluorescence was captured by the CCD camera every second for the first minute following the agonist addition with additional readings every 5 seconds for a total time period of 5 minutes. These images were digitally transferred to an interfaced PC and change in fluorescence intensity processed for each well. The exposure setting of the camera was 0.4 sec with an f-stop setting of 2 microns. The percent maximal intensity relative to that induced by 100 µM nicotine was plotted against the concentration of (1S5S)-3-(5,6-Dichloro-3-pyridinyl)-3,6-diazabicyclo[3.2.0]heptane and an EC₅₀ value of 5.5 µM was calculated. Independent measurements of 100 µM nicotine (100%) and unloaded cells (0%) were performed on each plate of cells with an average range of 20,000 fluorescence units. (1S5S)-3-(5,6-Dichloro-3-pyridinyl)-3,6-diazabicyclo[3.2.0]heptane induced calcium efflux into IMR-32 cells with an EC₅₀ of 5.5 µM, with a maximum efficacy 73% that of nicotine.

The IMR-32 FLIPR assay, described herein, measures cation efflux that is mediated through the ganglionic-like nicotinic acetylcholine receptor (nAChR) subtype. Agents that facilitate cation efflux of the ganglionic nAChR subtype have been linked to side effect liabilty such as cardiovascular pressor effects. For example, epibatidine, a known nAChR agent with cardiovascular pressor liability, was determined to have an EC₅₀ of 24 nM and a maximal efficacy of 137% (compared to nicotine) in the IMR-32 FLIPR assay. Both the higher (less-potent) EC₅₀ and the lower efficacy measured for (1S,5S)-3-(5,6-dichloro-3-pyridinyl)-3,6-diazabicyclo[3.2.0]heptane demonstrate a reduced side effect liability for (1S5S)-3-(5,6-Dichloro-3-pyridinyl)-3,6-diazabicyclo[3.2.0]heptane as compared to epibatidine.

The analgesic effect and the IMR-32 activity of (1S,55)-3-(5,6-dichloro-3-pyridinyl)-3,6-diazabicyclo[3.2.0]heptane was compared to related analogs as illustrated in Table 1.

**Table 1**

| | Analgesic Effect ED₅₀ (µmol/Kg) | IMR-32 activity EC₅₀ (µM) | IMR-32 activity % efficacy |
|---|---|---|---|
| (1S,5S)-3-(5,6-dichloro-3-pyridinyl)-3,6-diazabicyclo[3.2.0]heptane | 1 | 5.5 | 73 |
| (1R,5R)-3-(5,6-dichloro-3-pyridinyl)-3,6-diazabicyclo[3.2.0]heptane | | 0.078 | 106 |
| (1S,5S)-3-(6-chloro-5-methyl-3-pyridinyl)-3,6-diazabicyclo[3.2.0]heptane | >19 | 3.4 | 94 |
| (1S,5S)-3-(5-methoxy-3-pyridinyl)-3,6-diazabicyclo[3.2.0]heptane | >19 | 3.8 | 147 |
| (1S,5S)-3-(3-pyridinyl)-3,6-diazabicyclo[3.2.0]heptane | 20 | 23.2 | 100 |
| (1S,5S)-3-(6-chloro-3-pyridinyl)-3,6-diazabicyclo[3.2.0]heptane | 11 | 1.4 | 102 . |
| 5-[(1S,5S)-3,6-diazabicyclo[3.2.0]hept-3-yl]nicotinonitrile | >19 | 19.9 | 85 |
| 2-bromo-5-[(1S,5S)-3,6-diazabicyclo[3.2.0]hept-3-yl]nicotinonitrile | >19 | 1.2 | 103 |
| (1S,5S)-3-(6-bromo-5-chloro-3-pyridinyl)-3,6-diazabicyclo[3.2.0]heptane | >19 | 1.4 | 81 |

The data in Table 1 demonstrates that, compared to related analogs, (1S,5S)-3-(5,6-dichloro-3-pyridinyl)-3,6-diazabicyclo[3.2.0]heptane is a potent analgesic with reduced side effect liability. The side effect potential of the 1R,5R enantiomer evidenced by its potency in the 1MR-32 FLIPR assay precluded it from being tested in the analgesic model.

The in vitro binding data, in vivo analgesic assay, and IMR-32 FLIPR assay demonstrates that (1S,5S)-3-(5,6-dichloro-3-pyridinyl)-3,6-diazabicyclo[3.2.0]heptane binds to the nicotinic acetylcholine receptor, is useful for treating pain, in particular neuropathic pain, and has a reduced side effect liability.

The ability of compounds to improve cognitive function was assessed using the spatial discrimination version of the Morris water maze (Decker et al., Eur. J. Pharmacol. 261:217-222 (1994). This test measures the ability of an animal to utilize the context of extramaze visual cues to learn the location of a platform that provides safe escape from the water. Normal animals exhibit improved performance in this task in daily testing over a five-day period, while animals with a scopolamine-induced cognitive deficit do not exhibit the learning and memory consolidation required for improved performance in this test.

Male, Long-Evans rats, 300-400g, obtained from Charles River laboratories were used in this study. During two daily habituation sessions, rats are trained to find a visible escape platform in a pool (180 cm diameter and 60 cm high) filled to a depth of 37 cm with water made opaque with powdered milk. Water temperature is maintained at 26 °C. On the second day of habituation training, latency to escape measures are obtained in order to assure that animals are assigned to groups without swim speed bias. For spatial discrimination training, two visible platforms, covered in aluminum foil, are present. The platforms remain in the same position (diagonal to each other) through 5 days of training. Only one of the platforms provides escape; the other, made of expanded polystyrene, will not support the animals' weight. Rats receive six trials/day, with start position changed from trial to trial. The number of contacts with the incorrect platform (errors) serves as the dependent variable.

A cognitive deficit, as measured by increased number of errors in the water maze test, is induced by i.p. administration of the muscarinic antagonist scopolamine HBr (0.3 mg/kg), dosed 15 min prior to each daily discrimination training session (over five days total). Administration of (IS,5S)-3-(5,6-Dichloro-3-pyridinyl)-3,6-diazabicyclo[3.2.0]heptane at doses in the range of about 0.001 to about 5 µmol/kg, 30 minutes prior to the test (15 minutes prior to scopolamine) reversed the cognitive deficit and normalized the performance of the animals in the water maze.

The Morris water maze indicates that (1S,5S)-3-(5,6-Dichloro-3-pyridinyl)-3,6-diazabicyclo[3.2.0]heptane has utility in disease states involving cognitive deficits including, but not limited to, Alzheimer's disease, memory dysfunction, Parkinson's disease, senile dementia, attention deficit hyperactivity disorder, schizophrenia, and other cognitive impairments.

It is to be understood that (1S,5S)-3-(5,6-Dichloro-3-pyridinyl)-3,6-diazabicyclo[3.2.0]heptane has utility in disease states involving cognitive deficits and can be used in combination with other pharmaceutically acceptable cognitive enhancing active compounds.

(1S,5S)-3-(5,6-Dichloro-3-pyridinyl)-3,6-diazabicyclo[3.2.0]heptane can be used to treat pain via nicotinic acetylcholine receptors and as further described by M. Williams and S.P. Arneric, "Beyond the Tobacco Debate: dissecting out the therapeutic potential of nicotine" Exp. Opin. Invest. Drugs 5(8):1035-1045 (1996); and S.P. Arneric, J. P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995).

Additionally, (1S,5S)-3-(5,6-dichioro-3-pyridinyl)-3,6-diazabicyclo[3.2.0]heptane is useful for ameliorating or preventing disorders affected by nicotinic acetylcholine receptors, such as Alzheimer's disease, Parkinson's disease, memory dysfunction, Tourette's syndrome, sleep disorders, attention deficit hyperactivity disorder, neurodegeneration, inflammation, neuroprotection, anxiety, depression, mania, schizophrenia, anorexia and other eating disorders, AIDS-induced dementia, epilepsy, urinary incontinence, substance abuse, smoking cessation and inflammatory bowel syndrome.

Compounds that bind to the nicotinic acetylcholine receptor can be used to treat Alzheimer's disease as described by M. Williams and S.P. Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); S.P. Arneric, M.W. Holladay, J.P. Sullivan, "Cholinergic channel modulators as a novel therapeutic strategy for Alzheimer's disease" Exp. Opin. Invest. Drugs, 5(1):79-100 (1996); J. Lindstrom, "Nicotinic Acetylchloline Receptors in Health and Disease" Molecular Neurobiology 15:193-222 (1997); and G.K. Lloyd, et al., "The potential of subtype selective neuronal nicotinic acetylcholine receptor agonists as therapeutic agents" Life Sciences 62(17/18):1601-1606 (1998).

Compounds that bind to the nicotinic acetylcholine receptor can be used to treat Parkinson's disease as described by M. Williams and S.P. Arneric, "Beyond the Tobacco Debate: dissecting out the therapeutic potential of nicotine" Exp. Opin. Invest. Drugs 5(8):1035-1045 (1996); J. Lindstrom, "Nicotinic Acetylchloline Receptors in Health and Disease" Molecular Neurobiology 15:193-222 (1997); and G.K. Lloyd, et al., "The potential of subtype selective neuronal nicotinic acetylcholine receptor agonists as therapeutic agents" Life Sciences 62(17/18):1601-1606 (1998).

Compounds that bind to the nicotinic acetylcholine receptor can be used to treat memory dysfunction as described by M. Williams and S.P. Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and J. Lindstrom, "Nicotinic Acetylchloline Receptors in Health and Disease" Molecular Neurobiology 15:193-222 (1997).

Compounds that bind to the nicotinic acetylcholine receptor can be used to treat Tourette's syndrome as described by M. Williams and S.P. Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and J. Lindstrom, "Nicotinic Acetylchloline Receptors in Health and Disease" Molecular Neurobiology 15:193-222 (1997).

Compounds that bind to the nicotinic acetylcholine receptor can be used to treat sleeping disorders as described by M. Williams and S.P. Arneric, "Beyond the Tobacco Debate: dissecting out the therapeutic potential of nicotine" Exp. Opin. Invest. Drugs 5(8):1035-1045 (1996).

Compounds that bind to the nicotinic acetylcholine receptor can be used to treat attention deficit hyperactivity disorder as described by M. Williams and S.P. Arneric, "Beyond the Tobacco Debate: dissecting out the therapeutic potential of nicotine" Exp. Opin. Invest. Drugs 5(8):1035-1045 (1996); and S.P. Arneric, M.W. Holladay, J.P. Sullivan, "Cholinergic channel modulators as a novel therapeutic strategy for Alzheimer's disease" Exp. Opin. Invest. Drugs 5(1):79-100 (1996).

Compounds that bind to the nicotinic acetylcholine receptor can be used to treat neurodegeneration and to provide neuroprotection as described by S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and S.P. Arneric, M.W. Holladay, J.P. Sullivan, "Cholinergic channel modulators as a novel therapeutic strategy for Alzheimer's disease" Exp. Opin. Invest. Drugs 5(1):79-100 (1996).

Compounds that bind to the nicotinic acetylcholine receptor can be used to treat inflammation as described by S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and S.P. Arneric, M.W. Holladay, J.P. Sullivan, "Cholinergic channel modulators as a novel therapeutic strategy for Alzheimer's disease" Exp. Opin. Invest. Drugs 5(1):79-100 (1996).

Compounds that bind to the nicotinic acetylcholine receptor can be used to treat amyotrophic lateral sclerosis as described by M. Williams and S.P Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and S.P. Arneric, M.W. Holladay, J.P. Sullivan, "Cholinergic channel modulators as a novel therapeutic strategy for Alzheimer's disease" Exp. Opin. Invest. Drugs 5(1):79-100 (1996).

Compounds that bind to the nicotinic acetylcholine receptor can be used to treat anxiety as described by M. Williams and S.P Arneric, "Beyond the Tobacco Debate: dissecting out the therapeutic potential of nicotine" Exp. Opin. Invest. Drugs 5(8): 1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and S.P. Arneric, M.W. Holladay, J.P. Sullivan, "Cholinergic channel modulators as a novel therapeutic strategy for Alzheimer's disease" Exp. Opin. Invest. Drugs 5(1):79-100 (1996).

Compounds that bind to the nicotinic acetylcholine receptor can be used to treat depression as described by S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995).

Compounds that bind to the nicotinic acetylcholine receptor can be used to treat mania and schizophrenia can be demonstrated by M. Williams and S.P Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and J. Lindstrom, "Nicotinic Acetylchloline Receptors in Health and Disease" Molecular Neurobiology 15:193-222 (1997).

Compounds that bind to the nicotinic acetylcholine receptor can be used to treat anorexia and other eating disorders as described by M. Williams and S.P Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and J. Lindstrom, "Nicotinic Acetylchloline Receptors in Health and Disease" Molecular Neurobiology 15:193-222 (1997).

Compounds that bind to the nicotinic acetylcholine receptor can be used to treat AIDS-induced dementia as described by M. Williams and S.P Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and J. Lindstrom, "Nicotinic Acetylchloline Receptors in Health and Disease" Molecular Neurobiology 15:193-222 (1997).

Compounds that bind to the nicotinic acetylcholine receptor can be used to treat epilepsy as described by M. Williams and S.P Arneric, "Beyond the Tobacco Debate: dissecting out the therapeutic potential of nicotine" Exp. Opin. Invest. Drugs 5(8):1035-1045 (1996); S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995); and J. Lindstrom, "Nicotinic Acetylchloline Receptors in Health and Disease" Molecular Neurobiology 15:193-222 (1997).

Compounds that bind to the nicotinic acetylcholine receptor can be used to treat urinary incontinence as described by M. Williams and S.P Arneric, "Beyond the Tobacco Debate: dissecting out the therapeutic potential of nicotine" Exp. Opin. Invest. Drugs 5(8):1035-1045 (1996).

Compounds that bind to the nicotinic acetylcholine receptor can be used to treat premenstrual syndrome can be demonstrated by M. Williams and S.P Arneric, "Beyond the Tobacco Debate: dissecting out the therapeutic potential of nicotine" Exp. Opin. Invest. Drugs 5(8):1035-1045 (1996); and S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995).

Compounds that bind to the nicotinic acetylcholine receptor can be used to treat substance abuse as described by M. Williams and S.P Arneric, "Beyond the Tobacco Debate: dissecting out the therapeutic potential of nicotine" Exp. Opin. Invest. Drugs 5(8):1035-1045 (1996); and S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995).

Compounds that bind to the nicotinic acetylcholine receptor can be used to treat smoking cessation as described by M. Williams and S.P Arneric, "Beyond the Tobacco Debate: dissecting out the therapeutic potential of nicotine" Exp. Opin. Invest. Drugs 5(8):1035-1045 (1996); and S.P. Arneric, J.P. Sullivan, M. Williams, "Neuronal nicotinic acetylcholine receptors. Novel targets for central nervous system theraputics" Psychopharmacology: The Fourth Generation of Progress. F.E. Bloom and D.J. Kupfer (Eds.), Raven Press, New York 95-109 (1995).

Compounds that bind to the nicotinic acetylcholine receptor can be used to treat inflammatory bowel syndrome. M. Williams and S.P Arneric, "Beyond the Tobacco Debate: dissecting out the therapeutic potential of nicotine" Exp. Opin. Invest. Drugs 5(8):1035-1045 (1996); and J. Lindstrom, "Nicotinic Acetylchloline Receptors in Health and Disease" Molecular Neurobiology 15:193-222 (1997).

The present invention also provides pharmaceutical compositions that comprise (1S,5S)-3-(5,6-dichloro-3-pyridinyl)-3,6-diazabicyclo[3.2.0]heptane. The pharmaceutical compositions comprise (1S,5S)-3-(5,6-dichloro-3-pyridinyl)-3,6-diazabicyclo[3.2.0]heptane formulated together with one or more non-toxic pharmaceutically acceptable carriers.

The pharmaceutical compositions of this invention can be administered to humans and other mammals orally, rectally, parenterally , intracisternally, intravaginally, topically (as by powders, ointments or drops), bucally or as an oral or nasal spray. The term "parenterally," as used herein, refers to modes of administration which include intravenous, intramuscular, intraperitoneal, intrasternal, subcutaneous and intraarticular injection and infusion.

The term "pharmaceutically acceptable carrier," as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such as propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form can be brought about by the inclusion of agents which delay absorption such as aluminum monostearate and gelatin.

In some cases, in order to prolong the effect of (1S,5S)-3-(5,6-dichloro-3-pyridinyl)-3,6-diazabicyclo[3.2.0]heptane, it is desirable to slow the absorption of the (1S,5S)-3-(5,6-dichloro-3-pyridinyl)-3,6-diazabicyclo[3.2.0]heptane from subcutaneous or intramuscular injection. This can be accomplished by the use of a liquid suspension of crystalline or amorphous material with poor water solubility. The rate of absorption of (1S,5S)-3-(5,6-dichloro-3-pyridinyl)-3,6-diazabicyclo[3.2.0]heptane then depends upon its rate of dissolution which, in turn, may depend upon crystal size and crystalline form. Alternatively, delayed absorption of a parenterally administered (1S,5S)-3-(5,6-dichloro-3-pyridinyl)-3,6-diazabicyclo[3.2.0]heptane is accomplished by dissolving or suspending (1S,5S)-3-(5,6-dichloro-3-pyridinyl)-3,6-diazabicyclo[3.2.0]heptane in an oil vehicle.

Injectable depot forms are made by forming microencapsule matrices of the drug in biodegradable polymers such as polylactide-polyglycolide. Depending upon the ratio of (1S,5S)-3-(5,6-dichloro-3-pyndinyl)-3,6-diazabicyclo[3.2.0]heptane to polymer and the nature of the particular polymer employed, the rate of (1S,5S)-3-(5,6-dichloro-3-pyridinyl)-3,6-diazabicyclo[3.2.0]heptane release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, (1S,5S)-3-(5,6-dichloro-3-pyridinyl)-3,6-diazabicyclo[3.2.0]heptane may be mixed with at least one inert, pharmaceutically acceptable carrier or excipient, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

(1S,5S)-3-(5,6-Dichloro-3-pyridinyl)-3,6-diazabicyclo[3.2.0]heptane can also be in micro-encapsulated form, if appropriate, with one or more of the above-mentioned carriers.

Liquid dosage forms for oral administration include pharmaceutically acceptable emulsions, solutions, suspensions, syrups and elixirs. In addition to (1S,5S)-3-(5,6-dichloro-3-pyridinyl)-3,6-diazabicyclo[3.2.0]heptane, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to (1S,5S)-3-(5,6-dichloro-3-pyridinyl)-3,6-diazabicyclo[3.2.0]heptane, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which can be prepared by mixing (1S,5S)-3-(5,6-dichloro-3-pyridinyl)-3,6-diazabicyclo[3.2.0]heptane with suitable non-irritating carriers or carriers such as cocoa butter, polyethylene glycol or a suppository wax which are solid at room temperature but liquid at body temperature and therefore melt in the rectum or vaginal cavity and release (1S,5S)-3-(5,6-dichloro-3-pyridinyl)-3,6-diazabicyclo[3.2.0]heptane.

(1S,5S)-3-(5,6-Dichloro-3-pyridinyl)-3,6-diazabicyclo[3.2.0]heptane can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to (1S,5S)-3-(5,6-dichloro-3-pyridinyl)-3,6-diazabicyclo[3.2.0]heptane, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Dosage forms for topical administration of (1S,5S)-3-(5,6-dichloro-3-pyridinyl)-3,6-diazabicyclo[3.2.0]heptane include powders, sprays, ointments and inhalants. (1S,5S)-3-(5,6-Dichloro-3-pyridinyi)-3,6-diazabicyclo[3.2.0]heptane may be mixed under sterile conditions with a pharmaceutically acceptable carrier and any needed preservatives, buffers or propellants which may be required. Opthalmic formulations, eye ointments, powders and solutions are also contemplated as being within the scope of this invention.

Actual dosage levels of (1S,5S)-3-(5,6-dichloro-3-pyridinyl)-3,6-diazabicyclo[3.2.0]heptane in the pharmaceutical compositions of this invention can be varied so as to obtain an amount of (1S,5S)-3-(5,6-dichloro-3-pyridinyl)-3,6-diazabicyclo[3.2.0]heptane which is effective to achieve the desired therapeutic response for a particular patient, compositions and mode of administration. The selected dosage level will depend upon the activity of (1S,5S)-3-(5,6-dichloro-3-pyridinyl)-3,6-diazabicyclo[3.2.0]heptane, the route of administration, the severity of the condition being treated and the condition and prior medical history of the patient being treated.

When used in the above or other treatments, a therapeutically effective amount of (1S,5S)-3-(5,6-dichloro-3-pyridinyl)-3,6-diazabicyclo[3.2.0]heptane can be employed in pure form or, where such forms exist, in pharmaceutically acceptable salt, ester or prodrug form. The phrase "therapeutically effective amount" of (1S,5S)-3-(5,6-dichloro-3-pyridinyl)-3,6-diazabicyclo[3.2.0]heptane means a sufficient amount of the compound to treat disorders, at a reasonable benefit/risk ratio applicable to any medical treatment. It will be understood, however, that the total daily usage of (1S,5S)-3-(5,6-dichloro-3-pyridinyl)-3,6-diazabicyclo[3.2.0]heptane and compositions of the present invention will be decided by the attending physician within the scope of sound medical judgement. The specific therapeutically effective dose level for any particular patient will depend upon a variety of factors including the disorder being treated and the severity of the disorder; activity of (1S,5S)-3-(5,6-dichloro-3-pyridinyl)-3,6-diazabicyclo[3.2.0]heptane; the specific composition employed; the age, body weight, general health, sex and diet of the patient; the time of administration, route of administration, and rate of excretion of (1S,5S)-3-(5,6-dichloro-3-pyridinyl)-3,6-diazabicyclo[3.2.0]heptane; the duration of the treatment; drugs used in combination or coincidental with (1S,5S)-3-(5,6-dichloro-3-pyridinyl)-3,6-diazabicyclo[3.2.0]heptane; and like factors well known in the medical aits.

The term "pharmaceutically acceptable salt," as used herein, means salts derived from inorganic or organic acids. The salts can be prepared in situ during the final isolation and purification of (1S,5S)-3-(5,6-dichloro-3-pyridinyl)-3,6-diazabicylo[3.2.0]heptane or separately by reacting the free base of (1S,5S)-3-(5,6-dichloro-3-pyridinyl)-3,6-diazabicyclo[3.2.0]heptane with an inorganic or organic acid. Representative acid addition salts include, but are not limited to, acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, dihydrochloride, hydrobromide, hydroiodide, 2-hydroxyethansulfonate (isethionate), lactate, maleate, fumarate, methanesulfonate, nicotinate, 2-naphthalenesulfonate, oxalate, pamoate, pectinate, persulfate, 3-phenylpropionate, picrate, pivalate, propionate, succinate, sulfate, (L) tartrate, bis((L) tartrate), (D) tartrate, bis((D) tartrate), (DL) tartrate, bis((DL) tartrate), thiocyanate, phosphate, glutamate, bicarbonate, p-toluenesulfonate, and undecanoate.

The term "pharmaceutically acceptable amide," as used herein, means amides of (1S,5S)-3-(5,6-dichloro-3-pyddlnyl)-3,6-dlazabicyclo[3.2.0]heptane which are, within the scope of sound medical judgement, suitable for use in contact with the tissues of humans and lower animals without undue toxicity, irritation, allergic response, and the like. Amides of (1S,5S)-3-(5,6-dichloro-3-pyridinyl)-3,6-diazabicyclo[3.2.0]heptane may be prepared according to conventional methods. Representative examples include, but are not limited to, (1R,5S)-6-acetyl-3-(5,6-dichloro-3-pyridinyl)-3,6-diazabicyclo[3.2.0]heptane and (1R,5S)-6-benzoyl-3-(5,6-dlchloro-3-pyddinyl)-3,6-diazabicyelo[3.2.0]heptane.

(1S,5S)-3-(5,6-Dichloro-3-pyridlnyl)-3,6-dlazabicyclo[3.2.0]heptane can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

The total daily dose of (1S,5S)-3-(5,6-dichloro-3-pyridinyl)-3,6-diazabicyclo[3.2.0]heptane administered to a human or lower animal may range from about 0.001 to about 1000 mg/kg/day. For purposes of oral administration, more preferable doses can be in the range of from about 0.1 to about 50 mg/kg/day. If desired, the effective daily dose can be divided into multiple doses for purposes of administration; consequently, single dose compositions may contain such amounts or submultiples thereof to make up the daily dose.

## Claims

1. (1S,5S)-3-(5,6-Dichloro-3-pyridinyl)-3,6-diazabicyclo[3.2.0]heptane or a pharmaceutically acceptable salt thereof.

2. (1S,5S)-3-(5,6-dichloro-3-pyridinyl)-3,6-diazabicyclo[3.2.0]heptane for use in treating a disorder associated with acetylcholine nicotinic receptors in a mammal by administering to a mammal a therapeutically effective amount of said (1S,5S)-3-(5,6-dichloro-3-pyridinyl)-3,6-diazabicyclo[3.2.0]heptane.

3. (1S,5S)-3-(5,6-dichloro-3-pyridinyl)-3,6-diazabicyclo[3.2.0]heptane or a pharmaceutically acceptable salt thereof for use in treating pain in a mammal by administering to a mammal a therapeutically effective amount of said (1S,5S)-3-(5,6-dichloro-3-pyridinyl)-3,6-diazabicyclo[3.2.0]heptane or said pharmaceutically acceptable salt thereof.

4. (1S,5S)-3-(5,6-dichloro-3-pyridinyl)-3,6-diazabicyclo[3.2.0]heptane or a pharmaceutically acceptable salt thereof for use in treating a cognitive deficit in a mammal by administering to a mammal a therapeutically effective amount of said (1S,5S)-3-(5,6-dichloro-3-pyridinyl)-3,6-diazabicyclo[3.2.0]heptane or said pharmaceutically acceptable salt thereof.

5. The (1S,5S)-3-(5,6-dichloro-3-pyridinyl)-3,6-diazabicyclo[3.2.0]heptane or the pharmaceutically acceptable salt thereof for the use according to claim 4 wherein the disorder is Alzheimer's disease, Parkinson's disease, memory dysfunction, Tourette's syndrome, sleep disorders, attention deficit hyperactivity disorder, neurodegeneration, inflammation, neuroprotection, anxiety, depression, mania, schizophrenia, anorexia and other eating disorders, AIDS-induced dementia, epilepsy, urinary incontinence, substance abuse, smoking cessation or inflammatory bowel syndrome.

6. The (1S,5S)-3-(5,6-dichloro-3-pyridinyl)-3,6-diazabicyclo[3.2.0]heptane or the pharmaceutically acceptable salt thereof for the use according to claim 5 wherein the disorder is Alzheimer's disease, Parkinson's disease, memory dysfunction, attention deficit hyperactivity disorder, or schizophrenia.

7. A pharmaceutical composition comprising a therapeutically effective amount of (1S,5S)-3-(5,6-dichloro-3-pyridinyl)-3,6-diazabicyclo[3.2.0]heptane or a pharmaceutically acceptable salt thereof, in combination with a pharmaceutically acceptable carrier.

8. (1S,5S)-3-(5,6-dichloro-3-pyridinyl)-3,6-diazabicyclo[3.2.0]heptane or a pharmaceutically acceptable salt thereof for use in treating pain in a mammal by administering to a mammal a therapeutically effective amount of said (1S,5S)-3-(5,6-dichloro-3-pyridinyl)-3,6-diazabicyclo[3.2.0]heptane or said pharmaceutically acceptable salt thereof, in combination with a non-steroid anti-inflammatory agent.

9. (1S,5S)-3-(5,6-dichloro-3-pyridinyl)-3,6-diazabicyclo[3.2.0]heptane or a pharmaceutically acceptable salt thereof for use in treating pain in a mammal by administering to a mammal a therapeutically effective amount of said (1S,55)-3-(5,6-dichloro-3-pyridinyl)-3,6-diazabicyclo[3.2.0]heptane or said pharmaceutically acceptable salt thereof, in combination with an opioid.

10. (1S,5S)-3-(5,6-dichloro-3-pyridinyl)-3,6-diazabicyclo[3.2.0]heptane or a pharmaceutically acceptable salt thereof for use in treating pain in a mammal by administering to a mammal a therapeutically effective amount of said (1S,5S)-3-(5,6-dichloro-3-pyridinyl)-3,6-diazabicyclo[3.2.0]heptane or said pharmaceutically acceptable salt thereof, in combination with a tricyclic antidepressant.

11. (1S,5S)-3-(5,6-dichloro-3-pyridinyl)-3,6-diazabicyclo[3.2.0]heptane or a pharmaceutically acceptable salt thereof for use in treating pain in a mammal by administering to a mammal a therapeutically effective amount of said (1S,5S)-3-(5,6-dichloro-3-pyridinyl)-3,6-diazabicyclo[3.2.0]heptane or said pharmaceutically acceptable salt thereof, in combination with an anticonvulsant.

## Patentansprüche

1. (1S,5S)-3-(5,6-Dichlor-3-pyridinyl)-3,6-diazabicyclo[3.2.0]heptan oder ein pharmazeutisch verträgliches Salz davon.

2. (1S,5S)-3-(5,6-Dichlor-3-pyridinyl)-3,6-diazabicyclo[3.2.0]heptan zur Verwendung in der Behandlung einer Störung assoziiert mit Acetylcholinnikotinrezeptoren bei einem Säugetier durch Verabreichen einer therapeutisch wirksamen Menge des (1S,5S)-3-(5,6-Dichlor-3-pyridinyl)-3,6-diazabicyclo[3.2.0]heptans an ein Säugetier.

3. (1S,5S)-3-(5,6-Dichlor-3-pyridinyl)-3,6-diazabicyclo[3.2.0]heptan oder ein pharmazeutisch verträgliches Salz davon zur Verwendung in der Behandlung von Schmerzen bei einem Säugetier durch Verabreichen einer therapeutisch wirksamen Menge des (1S,5S)-3-(5,6-Dichlor-3-pyridinyl)-3,6-diazabicyclo[3.2.0]heptans oder des pharmazeutisch verträglichen Salzes davon an ein Säugetier.

4. (1S,5S)-3-(5,6-Dichlor-3-pyridinyl)-3,6-diazabicyclo[3.2.0]heptan oder ein pharmazeutisch verträgliches Salz davon zur Verwendung der Behandlung eines kognitiven Defizits bei einem Säugetier durch Verabreichen einer therapeutisch wirksamen Menge des (1S,5S)-3-(5,6-Dichlor-3-pyridinyl)-3,6-diazabicyclo[3.2.0]heptans oder des pharmazeutisch verträglichen Salzes davon an ein Säugetier.

5. Das (1S,5S)-3-(5,6-Dichlor-3-pyridinyl)-3,6-diazabicyclo[3.2.0]heptan oder das pharmazeutisch verträgliche Salz davon zur Verwendung gemäß Anspruch 4, worin die Störung Alzheimerkrankheit, Parkinson'sche Krankheit, Gedächtnisstörung, Tourette-Syndrom, Schlafstörungen, Aufmerksamkeitsdefizit-Hyperaktivitätsstörung, Neurodegeneration, Entzündung, Neuroprotektion, Angst, Depression, Manie, Schizophrenie, Anorexie und andere Essstörungen, AIDS-induzierte Demenz, Epilepsie, Harninkontinenz, Missbrauch von Suchtmitteln, Raucherentwöhnung oder entzündliche Darmerkrankung ist.

6. Das (1S,5S)-3-(5,6-Dichlor-3-pyridinyl)-3,6-diazabicyclo[3.2.0]heptan oder das pharmazeutisch verträgliche Salz davon zur Verwendung gemäß Anspruch 5, worin die Störung Alzheimerkrankheit, Parkinson'sche Krankheit, Gedächtnisstörung, Aufmerksamkeitsdefizit-Hyperaktivitätsstörung oder Schizophrenie ist.

7. Eine pharmazeutische Zusammensetzung, die eine therapeutisch wirksame Menge von (1S,5S)-3-(5,6-Dichlor-3-pyridinyl)-3,6-diazabicyclo[3.2.0]heptan oder einem pharmazeutisch verträglichen Salz davon in Kombination mit einem pharmazeutisch verträglichen Träger umfasst.

8. (1S,5S)-3-(5,6-Dichlor-3-pyridinyl)-3,6-diazabicyclo[3.2.0]heptan oder ein pharmazeutisch verträgliches Salz davon zur Verwendung in der Behandlung von Schmerz bei einem Säugetier durch Verabreichen einer therapeutisch wirksamen Menge des (1S,5S)-3-(5,6-Dichlor-3-pyridinyl)-3,6-diazabicyclo[3.2.0]heptans oder des pharmazeutisch verträglichen Salzes davon an ein Säugetier, in Kombination mit einem nichtsteroidalen entzündunghemmendes Mittel".

9. (1S,5S)-3-(5,6-Dichlor-3-pyridinyl)-3,6-diazabicyclo[3.2.0]heptan oder ein pharmazeutisch verträgliches Salz davon zur Verwendung in der Behandlung von Schmerz bei einem Säugetier durch Verabreichen einer therapeutisch wirksamen Menge des (1S,5S)-3-(5,6-Dichlor-3-pyridinyl)-3,6-diazabicyclo[3.2.0]heptans oder des pharmazeutisch verträglichen Salzes davon an ein Säugetier, in Kombination mit einem Opioid.

10. (1S,5S)-3-(5,6-Dichlor-3-pyridinyl)-3,6-diazabicyclo[3.2.0]heptan oder ein pharmazeutisch verträgliches Salz davon zur Verwendung in der Behandlung von Schmerz bei einem Säugetier durch Verabreichen einer therapeutisch wirksamen Menge des (1S,5S)-3-(5,6-Dichlor-3-pyridinyl)-3,6-diazabicyclo[3.2.0]heptans oder des pharmazeutisch verträglichen Salzes davon an ein Säugetier, in Kombination mit einem trizyklischen Antidepressivum.

11. (1S,5S)-3-(5,6-Dichlor-3-pyridinyl)-3,6-diazabicyclo[3.2.0]heptan oder ein pharmazeutisch verträgliches Salz davon zur Verwendung in der Behandlung von Schmerz bei einem Säugetier durch Verabreichen einer therapeutisch wirksamen Menge des (1S,5S)-3-(5,6-Dichlor-3-pyridinyl)-3,6-diazabicyclo[3.2.0]heptans oder des pharmazeutisch verträglichen Salzes davon an ein Säugetier, in Kombination mit einem antikonvulsiven Mittel.

## Revendications

1. (1S,5S)-3-(5,6-dichloro-3-pyridinyl)-3,6-diazabicyclo[3.2.0]-heptane ou sel pharmaceutiquement acceptable de celui-ci.

2. (1S,S5)-3-(5,6-dichloro-3-pyridinyl)-3,6-diazabicydo[3,2.0]-heptane destiné à une utilisation dans le traitement d'un trouble associé aux récepteurs nicotiniques d'acétylcholine chez un mammifère par administration à un mammifère d'une quantité thérapeutiquement efficace dudit (1S,5S)-3-(5,6-dichloro-3-pyridinyl)-3,6-diazabicyclo[3.2.0]heptane.

3. (1S,55)-3-(5,6-dichloro-3-pyridinyl)-3,6-diazabicyclo[3.2.0]-heptane ou sel pharmaceutiquement acceptable de celui-ci destiné à une utilisation dans le traitement de la douleur chez un mammifère par administration à un mammifère d'une quantité thérapeutiquement efficace dudit (1S,5S)-3-(5,6-dichloro-3-pyridinyl)-3,6-diazabicyclo[3.2.0]heptane ou dudit sel pharmaceutiquement acceptable de celui-ci.

4. (1S,5S)-3-(5,6-dichtoro-3-pyridinyl)-3,6-diazabicyclo[3.2.0]-heptane ou sel pharmaceutiquement acceptable de relui-ci destiné à une utilisation dans le traitement d'un déficit cognitif chez un mammifère par administration à un mammifère d'une quantité thérapeutiquement efficace dudit (1S,5S)-3-(5,6-dichloro-3-pyridinyl)-3,6-diazabicyclo[3.2.0]heptane ou dudit sel pharmaceutiquement acceptable de celui-ci.

5. (1S,5S)-3-(5,6-dichloro-3-pyridinyl)-3,6-diazabicyclo[3.2.0]-heptane ou sel pharmaceutiquement acceptable de celui-ci destiné à une utilisation selon la revendication 4, dans laquelle le trouble est la maladie d'Alzheimer, la maladie de Parkinson, le dysfonctionnement de la mémoire, le syndrome de la Tourette, les troubles du sommeil, le trouble d'hyperactivité déficitaire de l'attention, la neurodégénérescence, l'inflammation, la neuroprotection, l'anxiété, la dépression, la manie, la schizophrénie, l'anorexie et les autres troubles alimentaires, la démence du SIDA, l'épilepsie, l'incontinence urinaire, l'usage de drogues, le sevrage tabagique ou le syndrome intestinal inflammatoire.

6. (1S,5S)-3-(5,6-dichloro-3-pyridinyl)-3,6-cliazabicycl[3.2.0]-heptane ou sel pharmaceutiquement acceptable de celui-ci destiné à une utilisation selon la revendication 5, dans laquelle la maladie est la maladie d'Alzheimer, la maladie de Parkinson, le dysfonctionnement de la mémoire, le trouble d'hyperactivité déficitaire de l'attention ou la schizophrénie.

7. Composition pharmaceutique comprenant une quantité thérapeutiquement efficace de (1S,5S)-3-(5,6-dichioro-3-pyridinyl)-3,6-diazabicyclo[3.2.0]heptane ou d'un sel pharmaceutiquement acceptable de celui-ci en combinaison avec un véhicule pharmaceutiquement acceptable.

8. (1S,55)-3-(5,6-dichloro-3-pyridinyl)-3,6-diazabicyclo[3,2,0]-heptane ou sel pharmaceutiquement acceptable de celui-ci destiné à une utilisation dans le traitement de la douleur chez un mammifère par administration à un mammifère d'une quantité thérapeutiquement efficace dudit (1S,SS)-3-(5,6-dichforo-3-pyridinyl)-3,6-diazabicyclo[3.2.0]heptane ou dudit sel pharmaceutiquement acceptable de celui-ci en combinaison avec un agent anti-inflammatoire non stéroïdien.

9. (1S,5S)-3-(5,6-dichloro-3-pyridinyl)-3,6-diazabicyclo[3.2.0]-heptane ou sel pharmaceutiquement acceptable de celui-ci destiné à une utilisation dans le traitement de la douleur chez un mammifère par administration à un mammifère d'une quantité thérapeutiquement efficace dudit (1S,5S)-3-(5,6-dichloro-3-pyridinyl)-3,6-diazabicloa[3.2.0]heptane ou dudit sel pharmaceutiquement acceptable de celui-ci en combinaison avec un opioïde.

10. (1S,55)-3-(5,6-dichloro-3-pyridinyl)-3,6-diazabicyclo[3.2.0]-heptane ou sel pharmaceutiquement acceptable de celui-ci destiné à une utilisation dans le traitement de la douleur chez un mammifère par administration à un mammifère d'une quantité thérapeutiquement efficace dudit (1S,5S)-3-(5,6-dichloro-3-pyridinyl)-.3,6-diazabicydo[3.2.0]heptane ou dudit sel pharmaceutiquement acceptable de celui-ci en combinaison avec un antidépresseur tricyclique.

11. (1S,5S)-3-(5,6-dichloro-3-pyridinyl)-3,6-diazabicyclo[3.2.0]-heptane ou sel pharmaceutiquement acceptable de celui-ci destiné à une utilisation dans le traitement de la douleur chez un mammifère par administration à un mammifère d'une quantité thérapeutiquement efficace dudit (1S,5S)-3-(5,6-dichloro-3-pyridinyl)-3,6-diazabicyclo[3.2.0]heptane ou dudit sel pharmaceutiquement acceptable de celui-ci en combinaison avec un anticonvulsivant.
